# FASCICULE DE BREVET EUROPEEN

(11) **EP 1 058 726 B1**
(45) Date de publication et mention de la délivrance du brevet: **16.08.2006**
(21) Numéro de dépôt: 99904937.2
(22) Date de dépôt: 23.02.1999
(51) Int. Cl.: C12N 15/00, C07K 14/705, G01N 33/50

(54) **NOUVELLE FAMILLE DE CANAUX POTASSIUM DE MAMMIFERES MECANOSENSIBLES ET ACTIVES PAR LES ACIDES GRAS POLYINSATURES ET LEUR UTILISATION NOTAMMENT POUR LE CRIBLAGE DE DROGUES**
FAMILIE VON MECHANO-EMPFINDLICHEN KALIUMKANÄLEN BEI SÄUGETIEREN, DIE DURCH POLYUNGESÄTTIGTE FETTSÄUREN AKTIVIERT WERDEN UND DEREN VERWENDUNGEN
NOVEL MECHANICALLY SENSITIVE MAMMAL POTASSIUM CHANNEL FAMILY ACTIVATED BY POLYUNSATURATED FATTY ACIDS AND THEIR USE PARTICULARLY FOR SCREENING MEDICINES

(30) Priorité: 05.03.1998 FR 9802725
(43) Date de publication de la demande: 13.12.2000
(73) Titulaire: CENTRE NATIONAL DE LA RECHERCHE SCIENTIFIQUE (CNRS), 75794 Paris Cedex 16 (FR)
(72) Inventeur: HONORE, Eric, 06160 Juan les Pins (FR); FINK, Michel, 94260 Fresne (FR); LAZDUNSKI, Michel, F-06000 Nice (FR); LESAGE, Florian, 06000 Nice (FR); DUPRAT, Fabrice, 06220 Vallauris (FR)
(74) Mandataire: Breese, Pierre
(86) Numéro de dépôt international: PCT/FR1999/000404
(87) Numéro de publication internationale: WO 1999/045108

(56) Documents cités:
- WO-A-96/03415
- FR-A- 2 744 730
- FINK M. ET AL.: "Cloning, functional expression an brain localization of a novel unconventional outward rectifier K+ channel" EMBO JOURNAL, vol. 15, 1996, pages 6854-6862, XP002085602 EYNSHAM, OXFORD GB
- KIM D.: "A mechanosentitive K+ channel in heart cells" JOURNAL OF GENERAL PHYSIOLOGY, vol. 100, no. 6, 1992, pages 1021-1040, XP002085599
- FINK M. ET AL.: "A neuronal two P domain K+ channel stimulated by arachidonic acid and polyunsaturated fatty acids" EMBO JOURNAL, vol. 17, no. 12, 15 juin 1998 (1998-06-15), pages 3297-3308, XP002085600 EYNSHAM, OXFORD GB
- PATEL A.J. ET AL.: "A mammalian two pore domain mechano-gated S-like K+ channel" EMBO JOURNAL, vol. 17, no. 15, 3 août 1998 (1998-08-03), pages 4283-4290, XP002085601 EYNSHAM, OXFORD GB

## Description

La présente invention concerne une nouvelle classe de canaux potassium mécanosensibles activés par les acides gras polyinsaturés. L'invention est basée sur la découverte d'un nouveau canal potassium, dénommé TRAAK pour TWICK-Related AA-Actived K⁺channel, mécanosensibles activés par les acides gras polyinsaturés et également par le riluzole qui est un agent neuroprotecteur. Les propriétés des canaux de la famille TRAAK ainsi que leur distribution tissulaire confère à ces canaux un rôle primordial dans le transport de potassium chez un grand nombre de types cellulaires.

Les canaux potassium sont des protéines ubiquitaires et leur exceptionnelle diversité fonctionnelle en font des candidats idéaux pour un grand nombre de processus biologiques. Ils interviennent notamment dans la régulation de l'excitabilité neuronale et musculaire, sur le rythme cardiaque et sur la sécrétion d'hormone. Trois types structuraux de canaux potassium ont été décrits chez les mammifères. Le premier est le type "Shaker" qui est composé de sous-unités ayant 6 segments transmembranaires et un domaine P qui est impliqué dans la formation du pore ionique. Le second est le type IRK à deux segments transmembranaires et un domaine P. Le troisième a été décrit plus récemment et correspond au type TWIK qui a quatre segments transmembranaires et deux domaines P. Trois canaux de ce type ont été identifiés : TWIK-1 (Fink, M. et al. EMBO J. 15, 6854-6862 (1996), Lesage, F. et al EMBO J. 15, 1004-1011 (1996) TREK-1 et TASK (Duprat, F. et al. EMBO J. 16, 5464-5471 (1997). En dépit d'une structure générale conservée, ils ont des séquences primaires peu similaires, puiqu'ils présentent entre 20 à 25 % d'identité en acide aminé.

La présente invention est fondée sur la découverte et le clonage d'un nouveau canal désigné TRAAK, membre de la famille des canaux TWIK. Le gène codant ce canal est plus particulièrement homologue au niveau de sa séquence d'acides aminés au canal TREK-1 avec lequel il présente 38% d'identité en acide aminé. Le présente invention est également fondée sur les propriétés électrophysiologiques uniques de ces deux canaux TREK-1 et TRAAK. En effet, ces canaux produisent tous les deux des courants sélectifs au potassium qui sont activés par une tension appliquée à la membrane cellulaire, canaux dits mécanosensibles, ou par l'application d'acides gras polyinsaturés, notamment l'acide arachidonique qui est un messager essentiel de la communication inter et intra-cellulaire et un important modulateur de l'excitabilité neuronale (Ordway, R. W., Singer, J.J. et Walsh, j. V. 14, 96-100 (1991), Bliss, T. V. P. et Collingridge, G. L. Nature 31-39 (1993), Piomelli, D. Curr. Opin. Cell. Biol. 5, 274-280 (1993), Meves, H. Prog. Neurobiol. 43, 175-186 (1994), Piomelli, D. Crit. Rev. Neurobiol. 8, 65-83 (1994). Ces canaux sont également ouverts par le riluzole qui est un agent neuroprotecteur (Malgouris, C. et al. j. Neurosci. 9, 3720-3727 (1989), Pratt, j. et al. Neurosci. Lett. 140, 225-230 (1992) utilisé en clinique pour prolonger la survie de malades atteints de sclérose latérale amyotrophique.

La mise en évidence de cette nouvelle classe de canaux potassium et l'expression hétérologue de ces canaux permet notamment de disposer de nouveaux moyens pour rechercher par criblage des drogues capables de moduler l'activité de ces canaux potassium et donc de prévenir ou de traiter des maladies impliquant ces canaux, comme l'épilepsie, les pathologies cardiaques (arythmies) et vasculaires, les neurodégénérescences, particulièrement celles qui sont associées aux ischémies et aux anoxies, les pathologies endocriniennes associées à des anomalies dans la sécrétion d'hormones, les pathologies musculaires.

La présente invention a donc pour objet une protéine purifiée constituant un canal potassium mécanosensible activé par les acides gras polyinsaturés notamment l'acide arachidonique et par le riluzole. Plus particulièrement, l'invention concerne la protéine constituant le canal TRAAK dont la séquence en acides aminés est représentée dans la liste de séquences en annexe sous le numéro SEQ ID No : 1 ou un dérivé fonctionnellement équivalent de cette protéine.

De tels dérivés sont ceux dont la séquence comprend une-modification et/ou une suppression et/ou une addition d'un ou plusieurs résidus d'acides aminés, dès lors que cette modification et/ou supression et/ou addition ne modifie pas les propriétés du canal TRAAK. De tels dérivés peuvent être analysés par l'homme du métier selon les techniques décrites dans les exemples donnés ci-après qui ont permis de mettre en évidence les propriétés biophysiques et pharmacologiques du canal TRAAK. Un tel dérivé est plus particulièrement le canal TREK-1 dont la séquence en acides aminés est représentée dans la liste de séquences en annexe sous le numéro SEQ ID No : 2.

Des anticorps poly ou monoclonaux dirigés contre au moins une protéine constituant un canal ionique de l'invention peuvent être préparés par les méthodes classiques décrites dans la littérature. Ces anticorps sont utiles pour rechercher la présence des canaux ioniques de l'invention dans différents tissus humains ou animaux, mais ils peuvent aussi trouver des applications dans le domaine thérapeutique pour inhiber ou activer *in vivo,* grâce à leur spécificité, un canal TRAAK et/ou ses dérivés.

La présente invention a aussi pour objet une molécule d'acide nucléique purifiée comprenant ou constituée par une séquence nucléique codant pour une protéine constituant un canal potassium mécanosensible activé par les acides gras polyinsaturés notamment l'acide arachidonique et par le riluzole. Plus particulièrement l'invention concerne une molécule d'acide nucléique comprenant au moins une séquence codant pour la protéine constituant le canal TRAAK dont la séquence en acides aminés est représentée dans la liste de séquences en annexe sous le numéro SEQ ID No:1 ou pour un dérivé fonctionnellement équivalent de cette protéine. Une molécule d'ADN comprenant la séquence codant pour la protéine TRAAK est représentée dans la liste de séquences en annexe sous le numéro SEQ ID NO:1 ou sa séquence complémentaire. Plus particulièrement, une telle séquence d'acide nucléique comprend la séquence comprise entre les nucléotides 284 et 1477 de SEQ ID No:1 ou sa séquence complémentaire.

L'invention concerne également un vecteur comprenant au moins une molécule d'acide nucléique précédente, avantageusement associée à des séquences de contrôle adaptés, ainsi qu'un procédé de production ou d'expression dans un hôte cellulaire d'une protéine constituant un canal ionique selon l'invention. La préparation de ces vecteurs ainsi que la production ou l'expression dans un hôte des canaux de l'invention peuvent être réalisées par les techniques de biologie moléculaire et de génie génétique bien connues de l'homme du métier.

A titre d'exemple, un procédé de production d'une protéine constituant un canal cationique selon l'invention consiste :
- à transférer une molécule d'acide nucléique de l'invention ou un vecteur contenant ladite molécule dans un hôte cellulaire,
- à cultiver ledit hôte cellulaire dans des conditions permettant la production de la protéine constituant le canal potassium,
- à isoler, par tous moyens appropriés les protéines constituant les canaux potassium de l'invention.

A titre d'exemple, un procédé d'expression d'un canal ionique selon l'invention consiste :
- à transférer une molécule d'acide nucléique de l'invention ou un vecteur contenant ladite molécule dans un hôte cellulaire,
- à cultiver ledit hôte cellulaire dans des conditions permettant l'expression des canaux potassium de l'invention.

L'hôte cellulaire mis en oeuvre dans les procédés précédents peut être choisi parmi les procaryotes ou les eucaryotes et notamment parmi les bactéries, les levures, les cellules de mammifères, de plantes ou d'insectes.

Le vecteur utilisé est choisi en fonction de l'hôte dans lequel il sera transféré; il peut s'agir de tout vecteur comme un plasmide.

L'invention concerne donc aussi les hôtes cellulaires et plus particulièrement les cellules transformés exprimant des canaux potassium présentant des propriétés et une structure du type de celles du canal TRAAK obtenues conformément aux procédés précédents. Ces cellules sont utiles pour le criblage de substances capables de moduler les courants des canaux TRAAK. Ce criblage est effectué en mettant en contact des quantités variables d'une substance à tester avec des cellules exprimant les canaux de l'invention, puis en mesurant, par tous moyens appropriés, les effets éventuels de ladite substance sur les courants potassium desdits canaux. Des techniques électrophysiologiques permettent également ces études et font aussi l'objet de la présente invention dès lors qu'elles mettent en oeuvre les canaux TRAAK ou leurs dérivés. Ce procédé de criblage permet d'identifier des drogues capables de moduler l'activité des canaux potassium de l'invention et donc susceptibles de prévenir ou de traiter des maladies impliquant ces canaux. Une substance chimique ou biologique capable de modifier les ,courants d'un canal potassium selon l'invention susceptible d'être utilisée pour la préparation d'un médicament utile pour prévenir ou traiter des maladies du coeur ou du système nerveux chez un sujet humain ou animal, comme les pathologies cardiaques (arythmies) et vasculaires, les neurodégénérescences, particulièrement celles qui sont associées aux ischémies et aux anoxies, les pathologies endocriniennes associées à des anomalies dans la sécrétion d'hormones, les pathologies musculaires

Une molécule d'acide nucléique codant pour une protéine constituant un canal TRAAK ou un dérivé de celui-ci, ou un vecteur comprenant cette molécule d'acide nucléique ou encore une cellule exprimant des canaux TRAAK, sont aussi utiles pour la préparation d'animaux transgéniques. Il peut s'agir d'animaux surexprimant lesdits canaux, mais surtout d'animaux dit "knock out", c'est à dire présentant une déficience en ces canaux; ces animaux transgéniques sont préparés par des méthodes connues de l'homme du métier, et permettent de disposer de modèles vivants pour l'étude de pathologies animales associées aux canaux TRAAK.

Ces animaux transgéniques de même que les hôtes cellulaires décrits précédemment sont utiles en tant que modèles pour l'étude de pathologies associées à ces canaux potassium mécanosensibles activés par les acides gras polyinsaturés soient parce qu'ils surexpriment les canaux potassium du type canal TRAAK, soit parce qu'ils présentent une déficience en ces canaux potassium.

En outre, une protéine constituant un canal ionique neuronal TRAAK peut être aussi utile pour la fabrication de médicaments destinés à traiter ou prévenir des pathologies impliquant ces canaux. L'invention concerne donc aussi les compositions pharmaceutiques comprenant comme principe actif au moins une de ces protéines éventuellement associée à un véhicule physiologiquement acceptable.

De même, les molécules d'acide nucléique de l'invention ou les cellules transformées par ladite molécule sont donc susceptibles d'être utilisées dans des stratégies de thérapie génique afin de compenser une déficience des canaux TRAAK au niveau de un ou plusieurs tissus d'un patient. L'invention concerne donc aussi un médicament comprenant des molécules d'acide nucléique de l'invention ou de cellules transformées par lesdites molécules pour le traitement de pathologie impliquant les canaux TRAAK et leurs dérivés.

D'autres avantages et caractéristiques de l'invention apparaitront à la lecture des exemples qui suivent rapportant le travail de recherche ayant mené à l'identification et à la caractérisation de ces canaux potassium mécanosensibles activés par les acides gras et où il sera fait référence aux séquences et dessins en annexe dans lesquels :

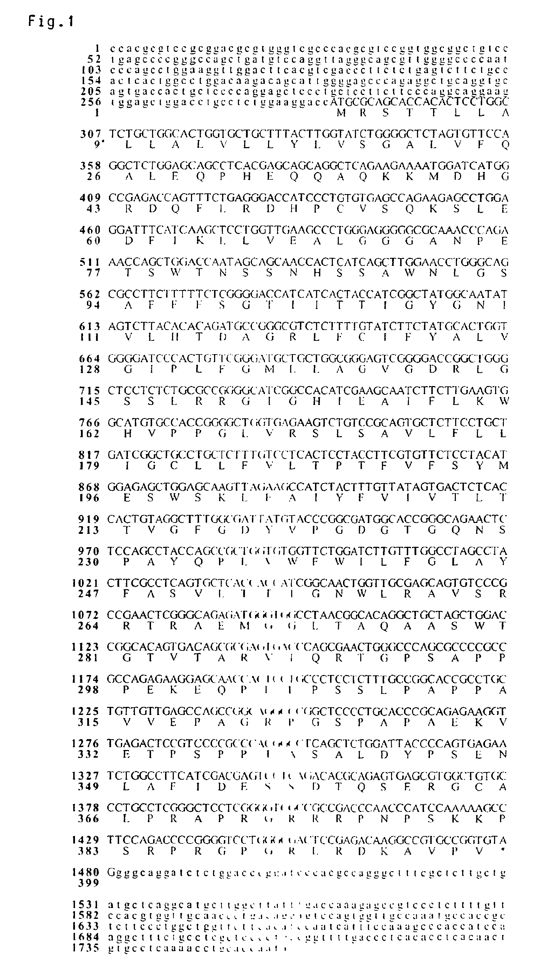

- la figure 1 et SEQ ID NO:1 représentent la séquence nucléotidique de l'ADNc de TRAAK et la séquence en acide aminé de la séquence codante.

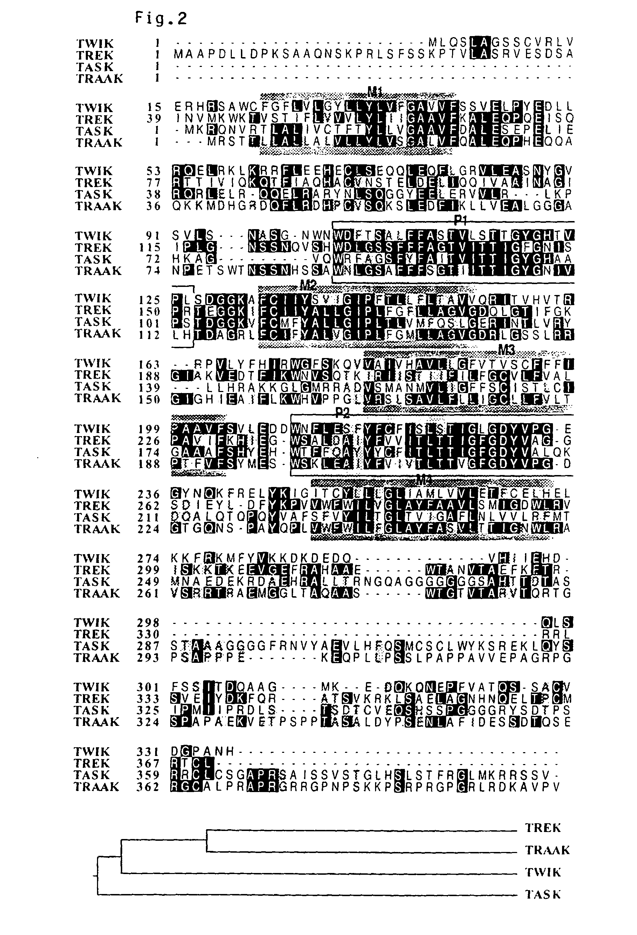

- la figure 2 représente l'alignement des séquences de TWIK-1, TREK-1, TASK et TRAAK qui sont les quatre canaux du type TWIK actuellement clonés chez les mammifères ainsi que le dendrogramme déduit de cet alignement. Les résidus identiques sont représentés sur fond noir et les résidus conservés sur fond gris.

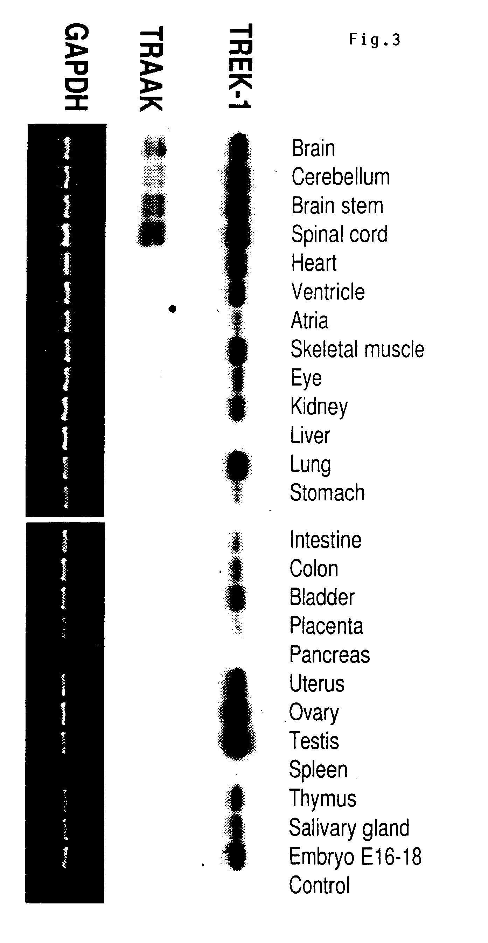

- la figure 3 représente l'analyse par RT-PCR de la distribution de TREK-1 et TRAAK dans les tissus de la souris adulte. Des fragments des transcripts codant TREK-1 et TRAAK ont été amplifiés par PCR à l'aide d'oligonucléotides spécifiques, transferés sur membrane de nylon puis sondés avec des oligonucléotides internes marqués au phosphore 32.

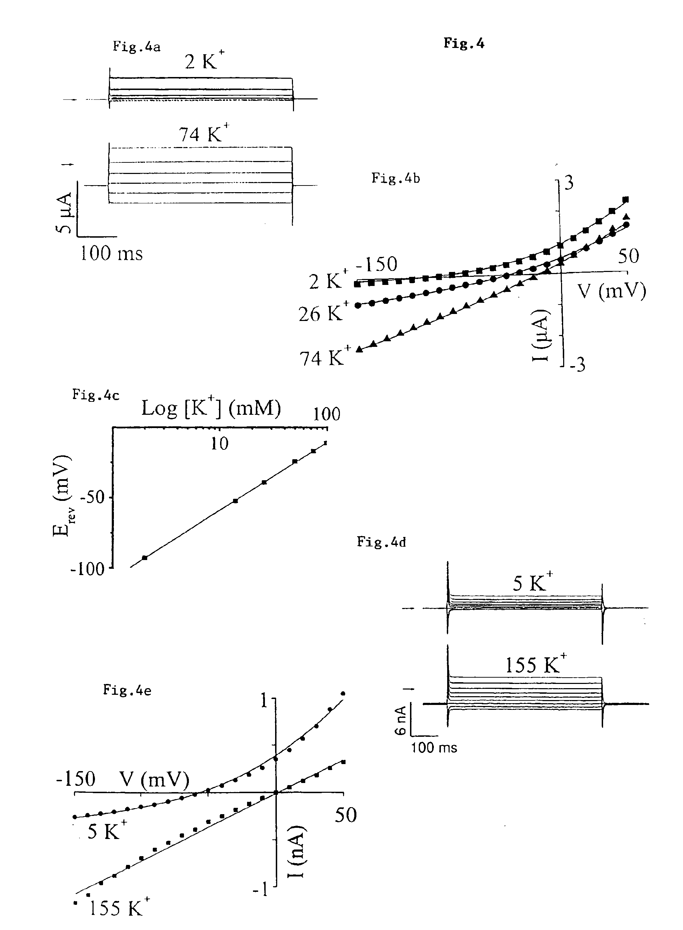

- la figure 4 montre les propriétés électrophysiologiques des courants TRAAK enregistrés par la technique de voltage imposé sur des ovocytes de Xénope ayant reçu une injection d'ARNc de TRAAK (a, b, c) et sur des cellules COS transfectés avec un vecteur exprimant TRAAK (d, e, f). En (a) : les ovocytes ont été maintenus à un potentiel de -80 mV puis les courants ont été enregistrés à la suite de sauts de potentiel de -150 à +50 mV par incrément de 20 mV. Les enregistrements ont été réalisés dans un milieu externe contenant une concentration en K⁺ de 2 mM ou de 74 mM. En (b) : relation courant-potentiel selon la même expérience qu'en (a). En (c) : renversement de potentiel (Eᵣₑᵥ) des courants TRAAK en fonction de la concentration externe en K⁺. En (d) : courants enregistrés sur des cellules COS transfectées par TRAAK suivant le même protocole qu'en (a). En (e) : relation courant-potentiel selon la même expérience qu'en (d).

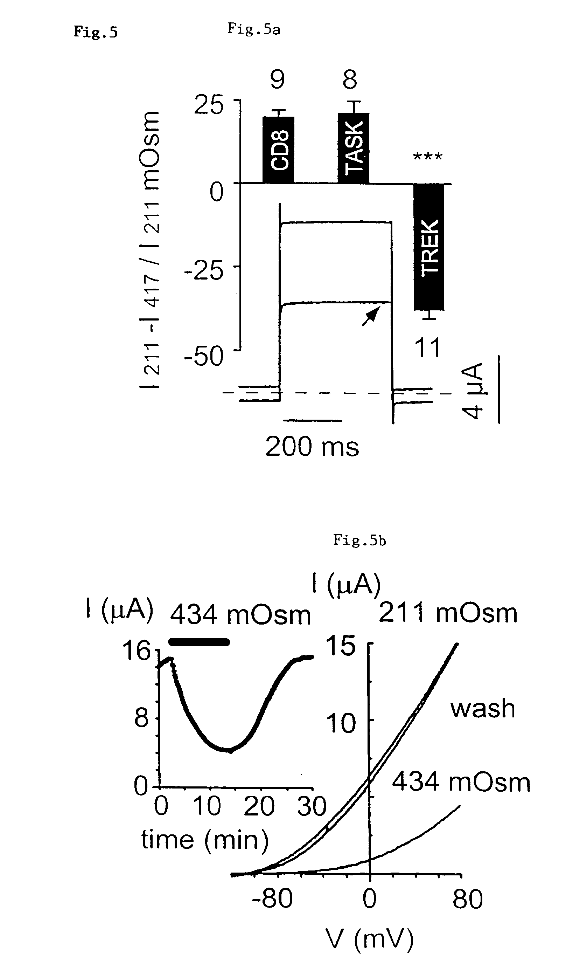

- la figure 5 montre l'effet de l'osmolarité du milieu externe sur des ovocytes ayant reçu une injection d'ARNc TREK-1 ou TASK. En (A) : comparaison des effets de l'application d'une solution hypertonique (417 mOsm, par addition de mannitol) sur des ovocytes témoins (CD8) et sur des ovocytes exprimant TASK ou TREK-1. Les courants sont mesurés après un saut de potentiel de -80 à +80 mV. En inset est montré le courant TREK-1 avant et après (indiqué par une flêche) l'application de la solution hypertonique. En (B) : effet réversible d'une solution hypertonique (434 mOsm, par addition de sucrose) sur les relations courant-potentiel déduites de rampes de potentiel qui durent 600 msec. En inset est montré la cinétique de l'effet produit par la solution hypertonique. Les courants sont mesurés à 80mV.

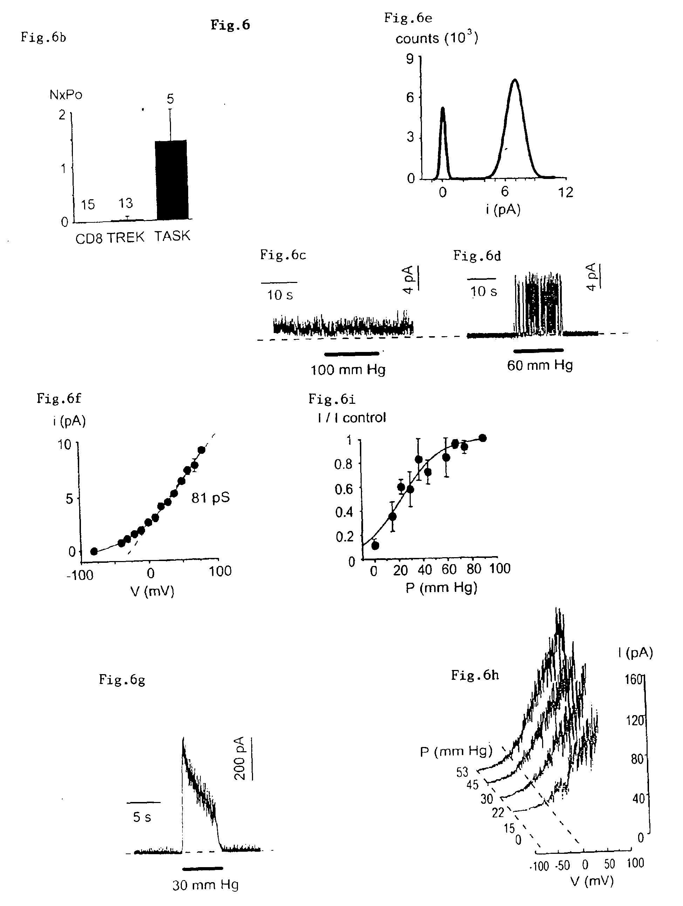

- la figure 6 montre que TREK-1 est un canal potassium mécanosensible dans les cellules COS transfectées. En (B) : activités canal (N*Po) dans des "patches" de membrane maintenus à 0 mV et obtenus dans la configuration cellule attachée à partir de cellules témoins (CD8), ou de cellules transfectées par TREK-1 et TASK. En (C) l'étirement de la membrane n'a pas d'effet sur l'activité du canal TASK (configuration cellule attachée). Le "patch" est maintenu à 50mV. En (D) : les canaux TREK-1 sont silencieux au repos et ouvert lors d'une tension de la membrane. Le "patch" est maintenu à +50mV. En (E) histogramme donnant l'amplitude de l'activité canal engendrée par la tension de la membrane et illustrée en (G). En (F) : relation courant-potentiel en canal unique de TREK-1 (n=6). La conductance de 81 pS a été calculée entre 0 et 80 mV. En (G) : activation de TREK-1 par étirement de la membrane (30 mm Hg) dans la configuration "inside-out". Le potentiel de maintien est 100 mV. En (H) : effet produits par des tensions de plus en plus importantes (5 sec de durée) sur la relation courant-potentiel d'un "patch" exprimant TREK-1. En (I) : courbe dose-effet de l'activation de TREK-1 par la tension (n=6). La courbe est tracée en suivant les points expérimentaux suivant la relation de Boltzmann.

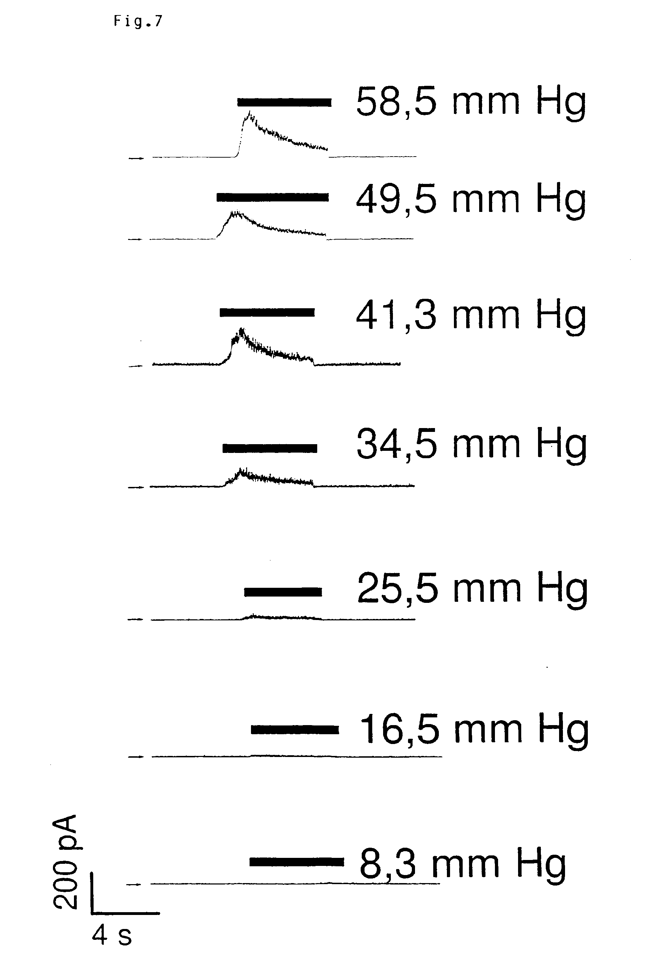

- la figure 7 montre l'activation de TRAAK par l'étirement de la membrane cellulaire dans les cellules COS transfectées. Le courant est enregistré à 0 mV dans la configuration "inside-out". Les dépressions appliquées via la pipette d'enregistrement sont indiquées sur la droite des traces.

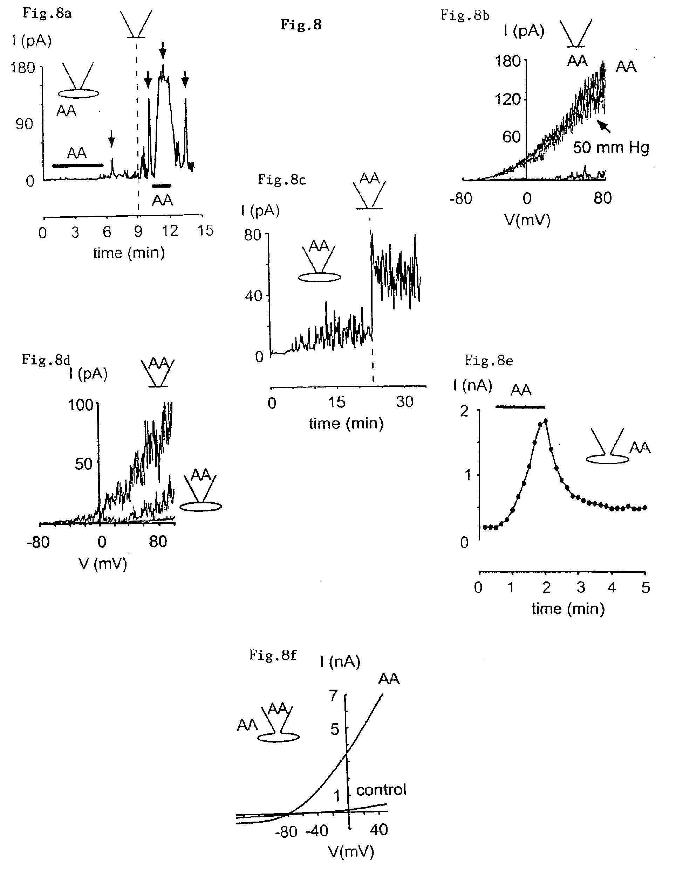

- la figure 8 montre l'activation de TREK-1 par l'acide arachidonique dans les cellules COS transfectées. En (A) : l'activité de TREK-1 est enregistrée dans la configuration cellule attachée. Le "patch" est stimulé par une rampe de potentiel durant 800 msec toutes les 5 sec. Les courants sont mesurés à 80 mV. Les applications d'acide arachidonique (AA, 10µM) sont indiquées par les barres horizontales. Au cours de l'expérience, le "patch" a été stimulé par des tensions de 50 mm Hg (indiquées par des flèches). A 9 min, le "patch" a été excisé dans la configuration "inside-out". En (B) : relations courant-potentiel qui correspondent à l'expérience illustrée en (A). En (C) : activité de TREK-1 dans la configuration cellule attachée avec 10 µM AA dans la pipette. La rampe de potentiel dure 800 msec et les courants sont mesurés à 80mV. En (D) : relations courant-potentiel en canal unique au moment où la pipette est posée sur la membrane ou après 20 min et 1 min après avoir excisé le "patch" dans la configuration "inside-out". En (E) effet de l'AA (10µM) sur le courant TREK-1 enregistré en cellule entière. Le courant est mesuré à 80mV. En (F) : l'AA est sans effet sur le courant TREK-1 mesuré en cellule entière lorsqu'il est dans la pipette. Le courant est mesuré 30 min après avoir rompu le "patch" (trace contrôle) par une rampe de potentiel de 800 msec. Le courant est ensuite mesuré après une application d'AA de 1 min dans le milieu externe (trace AA).

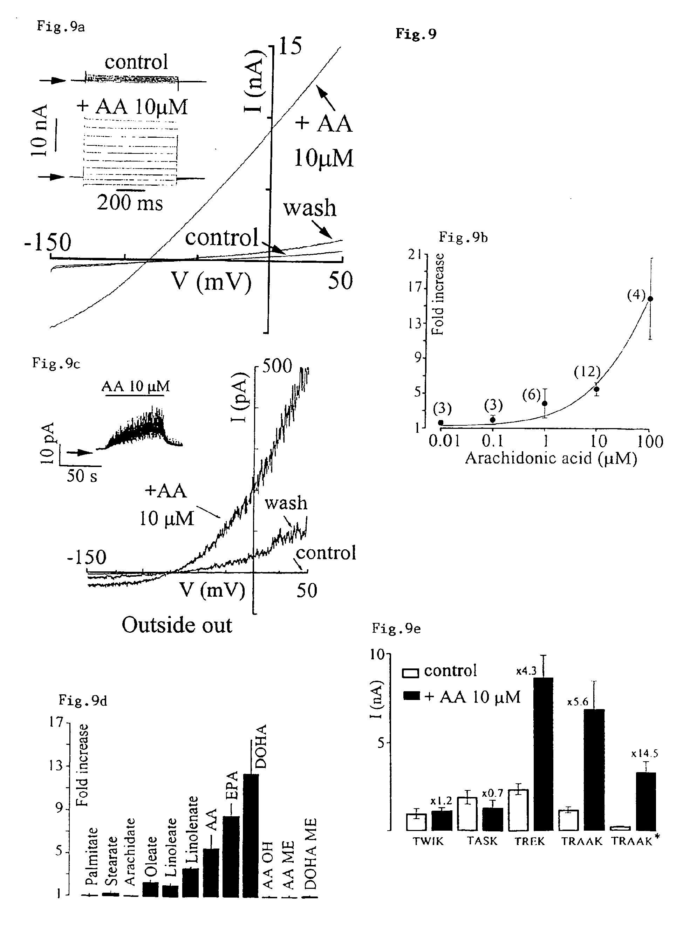

- la figure 9 montre l'effet de l'acide arachidonique et d'autres acides gras sur le canal TRAAK exprimé dans des cellules COS transfectées. En (a) : relations courant-potentiel obtenues à partir de rampes de potentiel de 500 msec allant de -150 à +50 mV, après application d'AA (10 µM) et après lavage. En inset sont représentés les courants déclenchés par des sauts de potentiel de -130 à +50 mV par incrément de 20 mV. Le potentiel de maintien est -80mV. En (b) : relation dose-effet de l'activation de TRAAK par l'AA. En (c) : relations courant-potentiel obtenus comme en (a) dans la configuration "outside-out". En inset est montré l'effet de l'AA à 20 mV . En (e) : histogramme représentant le coefficient d'augmentation des courants obtenus après application de différents acides gras (10µM). En (f) : histogramme montant la valeur des courants enregistrés dans la configuration de la cellule entière avant et après application d'AA sur des cellules transfectées transitoirement par TWIK-1, TASK, TREK-1 et TRAAK et sur des cellules transfectées de façon stable par TRAAK. Le coefficient d'augmentation est indiqué dans chaque cas.

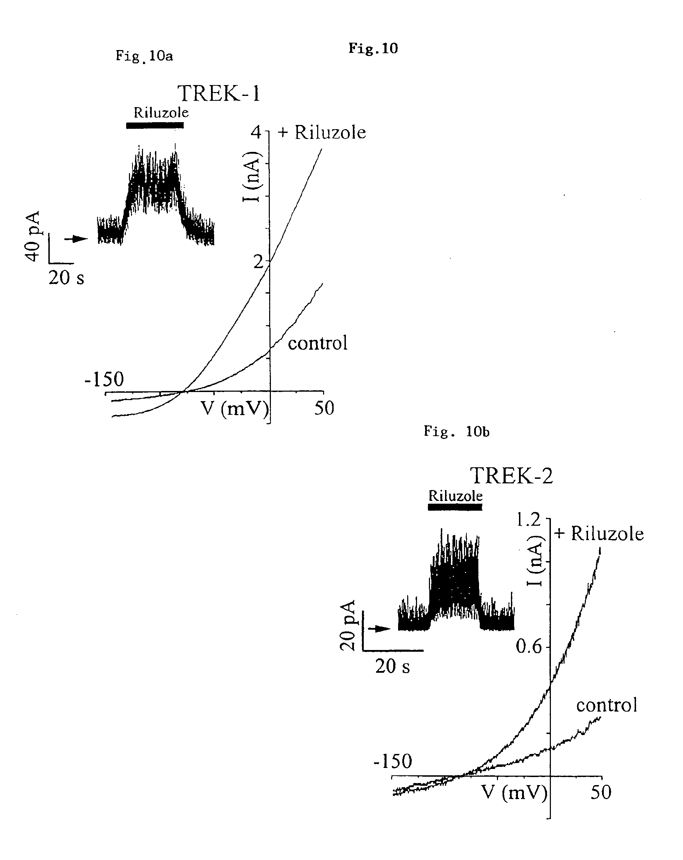

- la figure 10 montre l'effet du riluzole sur les courants TREK-1 et TRAAK désigné TREK-2. Les relations courant-potentiel sont obtenus comme dans la figure 9a avant et après l'application de riluzole (100µM) sur des cellules COS transfectées. En inset sont montrés les effets du riluzole sur les courants enregistrés dans la configuration "outside-out".

### I - Clonage, structure primaire et distribution tissulaire de TRAAK.

La séquence du canal TWIK-1 a été utilisée pour rechercher des séquences homologues dans les banques publiques de données d'ADN (Genbank et EMBL) en mettant en oeuvre le programme d'alignement BLAST. Il a ainsi été identifié une séquence exprimée TAG humaine qui a servi à cribler une banque d'ADNc de cerveau de souris. Plusieurs clones ont été isolés et caractérisés. Le plus long a été séquencé. Les caractéristiques suivantes ont été mises en évidence :
- les ADNc isolés contiennent une phase ouverte de lecture de 1197 nucléotides codant pour un polypeptide de 398 résidus. Les séquences nucléotidiques et protéiques sont montrées dans la figure 1.
- cette protéine contient 4 segments transmembranaires potentiels et deux domaines P. Elle possède donc la même structure générale que les canaux TWIK-1, TREK-1 et TASK. De plus, elle présente des homologies de séquence avec ces canaux : environ 20-25% d'identité avec TWIK-1 et TASK et 38% avec TREK-1. En dehors des domaines P qui sont présents dans tous les canaux potassium clonés, elle n'a pas d'homologie de séquence significative avec les canaux de type *Shaker* et IRK. Elle appartient donc à la famille TWIK-1 et son homologue le plus proche est TREK-1. Ces relations apparaissent dans la figure 2 au niveau de l'alignement des séquences protéiques ainsi que dans le dendrogramme qui est déduit de cet alignement. TRAAK et TREK-1 forment donc une sous-classe structurale au sein de la famille TWIK-1.
- les séquences de différents oligonucléotides ont été déduits à partir de la séquence de TRAAK. Ces oligonucléotides ont permis par RT-PCR d'étudier la distribution du transcrit codant TRAAK dans les tissus de souris adulte. Comme le montre la figure 3, TRAAK est exclusivement exprimé dans des tissus neuronaux : cerveau, cervelet, moelle épinière et rétine. Cette distribution est très différente de celle de son plus proche homologue qui est le canal TREK-1. Celui a une distribution quasi ubiquitaire et est présent aussi bien dans les tissus excitables que dans les tissus non-excitables.

### II - Expression fonctionnelle de TRAAK.

Pour l'étude fonctionnelle, la séquence codante de TRAAK a été insérée dans le vecteur pEXO et un ARN complémentaire (ARNc) a été synthétisé à partir de cette construction et injecté dans des ovocytes de Xénope. Pour l'expression dans les cellules COS, la séquence de TRAAK a été sous-clonée dans un vecteur d'expression sous le contrôle d'un promoteur eucaryote et transfectée dans les cellules. Un courant non-inactivant absent des ovocytes et des cellules témoins a été mesuré par la technique de potentiel imposé comme représenté à la figure 4. L'activation est instantané et ne peut être résolue car elle est masquée par la décharge capacitive du courant enregistré au début du saut de potentiel. La relation courant-potentiel rectifie dans le sens sortant lorsque la concentration externe en K⁺ est égale à 2 mM. Des courants entrants sont observés lorsque la concentration externe en K⁺ est augmentée. Quelque soit cette concentration, les courbes courant-potentiel suivent parfaitement la relation de Goldman-Hodgkin-Katz. Cela démontre que les courants TRAAK n'ont pas de rectification autre que celle qui est due aux concentrations dissymétriques de K⁺ de chaque côté de la membrane et que TRAAK est un canal qui n'est pas dépendant du potentiel. Le canal TRAAK est sélectif au potassium. Le renversement du potentiel des courants suit le potentiel d'équilibre du K⁺ et le changement par 10 de la concentration en K⁺ conduit à un changement de la valeur d'inversion du potentiel conforme à celle prédite par l'équation de Nernst (48.7+-0.7 mV par 10, n=4).

Les propriétés de TRAAK, absence de cinétiques d'activation et d'inactivation aussi bien que son ouverture à tous les potentiels de membrane, sont des caractéristiques des canaux potassium dits de fuite. Comme prévu pour des canaux de ce type, son expression dans les oocytes est associée à une forte polarisation. Le potentiel de repos de la membrane passe de -43±2,4 mV, (n=7), dans les oocytes de contrôle à -88±1,4mV, (n=23)dans les oocytes transfectés, une valeur proche du potentiel d'équilibre du potassium. TRAAK a été aussi exprimé dans les cellules COS-M6 transfectées. Dans ce système aussi, les courants TRAAK sont instantanés et ne s'inactivent pas. L'enregistrement des "patch" en configuration "outside-out" indique une conductance unitaire de TRAAK égale à 45,5 ± 3,7 pS (n = 10).

### III - TREK-1 et TRAAK sont des canaux mécanosensibles.

Il a été établi que la sous-classe structurale formée par les canaux K⁺ TREK-1 et TRAAK est associée à des propriétés électrophysiologiques uniques parmi les canaux K⁺ de type TWIK. Les canaux TREK-1 et TRAAK sont en effet activés par une tension appliquée à la membrane plasmique. Cette tension est obtenue soit indirectement en changeant l'osmolarité du milieu externe et donc le volume de la cellule soit plus directement en appliquant une dépression dans la pipette d'enregistrement. Les caractéristiques suivantes ont été mises en évidence :
- la figure 5 démontre que l'expression du canal TREK-1 dans des ovocytes de Xénope qui sont maintenus dans un milieu hypotonique induit des courants instantanés et non-inactivants. Quand l'osmolarité du milieu externe est augmentée en y ajoutant du mannitol, une importante diminution de l'amplitude du courant TREK-1 est observée ce qui démontre une sensibilité du canal au volume cellulaire. Le canal TASK lui n'est pas affecté par l'osmolarité du milieu externe.
- la figure 6 démontre que le canal TREK-1 est mécanosensible. Dans des cellules COS transfectées et sous des conditions de repos, l'activité de TREK-1 est indétectable dans la configuration cellule attachée alors que l'activité de TASK est facilement mesurable dans les mêmes conditions. Cependant, une dépression appliquée à la membrane par l'intermédiaire de la pipette d'enregistrement déclenche une ouverture du canal TREK-1. Un tel effet n'est pas vu avec TASK. L'activation de TREK-1 induit par la tension est également obtenu dans la configuration "inside-out" c'est à dire lorsque le "patch" est excisé et que la face interne de la membrane se retrouve en contact avec le milieu externe. Dans cette configuration, l'activité du canal est également absente ou très faible si on n'applique pas de tension à la membrane. L'effet de la tension est graduée et une activation qui est égale à la moitié de la valeur maximale est détectée pour une dépression équivalente à 23 mm de mercure. D'autre part, la figure 6h montre que l'activation induite par l'étirement est indépendante du potentiel de membrane.
- la figure 7 montre également que TRAAK est un canal activé par l'étirement. En absence de dépression ou pour de faibles valeurs, le canal TRAAK est inactif. Pour des valeurs plus élevées, le canal est activé et un courant est enregistré. Durant l'application de la dépression, une diminution de l'activité du canal est observable comme dans le cas de TREK-1.

### IV - TREK-1 et TRAAK sont activés par l'acide arachidonique et d'autres acides gras polyinsaturés.

L'activation des canaux TREK-1 et TRAAK par étirement mécanique de la membrane est mimée par l'application d'acide arachidonique et par l'application d'autres acides gras polyinsaturés, mais pas par l'application d'acides gras saturés. Les caractéristiques suivantes ont été mises en évidence :
- la figure 8 démontre que TREK-1 est activé par l'acide arachidonique (AA). L'application d'AA sur des cellules témoins (CD8) n'a pas d'effet. Les activations obtenues par étirement de la membrane et par application d'AA sont similaires en amplitude mais ne sont pas additives. Les deux types d'activation sont réprimées dans la configuration cellule attachée. Quand la pipette d'enregistrement contient de l'AA, l'excision du "patch" dans la configuration "inside-out" induit de façon reproductible une augmentation importante de l'activité de TREK-1. De la même manière, l'amplitude de l'activation induite par une dépression appliquée dans la pipette d'enregistrement est plus importante lorsque le "patch" est excisé. Finalement, il a été observé qu'en cellule entière, l'AA interne n'active pas TREK-1. Quand la cellule est dialysée pour des périodes aussi longues que 30 minutes, aucune activation du canal par l'AA interne n'a lieu bien que l'activation soit observée quelques secondes après l'application d'AA dans le milieu externe. Ces résultats indiquent que l'AA active TREK-1 seulement lorsqu'il est appliqué sur la face externe de la membrane.
- la figure 9 démontre que le canal TRAAK est activé par l'AA de la même manière que TREK-1. L'activation est réversible et dépendante de la concentration appliquée. Cette activation est aussi observée dans la configuration "outside -out". L'activation de TRAAK par l'AA n'est pas prévenue quand la perfusion d'AA contient un mélange d'inhibiteurs du métabolisme de l'AA (acide nordihydroguaiaretique pour la lipoxygénase, l'indomethacine pour la cyclooxygénase, clotrimazole pour époxygénase et l'ETYA qui inhibe l'ensemble des voies de métabolisation de l'AA, tous à 10mM). Dans ces conditions, l'augmentation du courant induit par AA est de 6.6+-0,5 fois (n=3)(à +50mV). Une augmentation de 1.7±0.4 fois (n=3) du courant de potassium de fond peut être observé après l'administration d'un coktail d'inhibiteurs en l'absence d'AA Ce résultat démontre que l'activation par l'AA ne requière pas la transformation de l'AA en eicosanoïdes.
- la figure 9 démontre également que des acides gras autres que l'AA activent le canal. Cette activation est spécifique des acides gras *cis* polyinsaturés et est observée avec les acides oléique (C18Δ9), linoléique (C18Δ9,12), linolénique (C18Δ9,12,15), eicosapentaenoique (EPA, C20Δ5,8,11,14,17) et docosohexaenoiques (DOHA, C20Δ4,7,10,13,16,19) à une concentration de 10 mM. Les acides saturés tels que les acides palmitique (C16), stéarique (C18) et arachidique (C20) sont quant à eux sans effet. Les dérivés de l'AA et de l'acide docosohexaenoique où la fonction carboxylique est substituée par une fonction alcool (AA-OH) ou methyl ester (AA-ME, DOHA-ME) sont également inactifs sur TRAAK. L'effet de l'AA sur TRAAK est observable aussi bien sur des cellules transfectées de façon transitoire que de façon stable (3 lignées de cellules stables indépendantes ont été testées).
- finalement, la figure 9 démontre que l'effet d'activation par l'AA est spécifique de TREK-1 et TRAAK. Aucun effet du même type n'est observé sur les canaux TWIK-1 et TASK.

Dans les oocytes, TRAAK est insensible aux agents bloquant des canaux potassium classiques tels que le tétraéthylammonium (TEA, 1mM), la 4-aminopyridine (4-AP, 1mM) et la quinine (100 mM). Inversement, Ba²⁺ (1mM) bloque 56,7 ± 4,6 %, n=5 du courant TRAAK à +40 mV.

### V - Les canaux TREK-1 et TRAAK sont activés par un agent neuroprotecteur le riluzole.

Le riluzole est un agent neuroprotecteur qui est utilisé pour prolonger la survie des malades atteint de sclérose latérale amyotrophique. Le figure 10 démontre que cet agent pharmacologique est un ouvreur des canaux TREK-1 et TRAAK. TREK-1 et TRAAK sont les premiers canaux ioniques dont l'activité est stimulée par le riluzole.

## Revendications

1. Protéine purifiée constituant un canal potassium mécanosensible activé par les acides gras polyinsaturés notamment l'acide arachidonique et par le riluzole, dont la séquence en acides aminés est représentée dans la liste de séquences en annexe sous le numéro SEQ ID No:1.

2. Anticorps poly ou monoclonaux dirigés contre au moins une protéine constituant un canal ionique selon la revendication 1.

3. Molécule d'acide nucléique purifiée comprenant ou constituée par une séquence nucléique codant pour une protéine selon la revendication 1.

4. Molécule d'acide nucléique selon la revendication 3 comprenant la séquence comprise entre les nucléotides 284 et 1477 de la séquence représentée dans la liste de séquences en annexe sous le numéro SEQ ID NO:1 ou sa séquence complémentaire.

5. Vecteur comprenant au moins une molécule d'acide nucléique selon l'une quelconque des revendications 3 ou 4.

6. Procédé de production d'une protéine constituant un canal potassium selon la revendication 1, **caractérisé en ce qu'**il consiste :
- à transférer une molécule d'acide nucléique selon l'une des revendications 3 ou 4 ou un vecteur selon la revendication 5 dans un hôte cellulaire,
- à cultiver ledit hôte cellulaire dans des conditions permettant la production de la protéine constituant ledit canal potassium,
- à isoler, par tous moyens appropriés les protéines constituant lesdits canaux.

7. Procédé d'expression d'un canal potassium selon la revendication 1, **caractérisé en ce qu'**il consiste :
- à transférer une molécule d'acide nucléique selon l'une des revendications 3 ou 4 ou un vecteur selon la revendication 5 dans un hôte cellulaire,
- à cultiver ledit hôte cellulaire dans des conditions permettant la production desdits canaux potassium.

8. Hôte cellulaire non humain susceptible d'être obtenu par un procédé selon la revendication 7.

9. Procédé de criblage de substances capables de moduler l'activité de canaux potassium mécanosensibles activés par les acides gras polyinsaturés selon la revendication 1, **caractérisé en ce que** l'on met en contact des quantités variables d'une substance à tester avec des cellules selon la revendication 7, puis l'on mesure, par tous moyens appropriés, les effets éventuels de ladite substance sur les courants desdits canaux.

10. Procédé selon la revendication 9 appliqué au criblage de substances capables de prévenir ou traiter des maladies du système nerveux chez un sujet humain ou animal.

11. Procédé selon la revendication 10 appliqué au criblage de substances capables de prévenir ou traiter les neurodégénérescences.

12. Procédé selon la revendication 11 appliqué au criblage de substances capables de prévenir ou traiter les neurodégénérescences associées aux ischémies et aux anoxies.

13. Composition pharmaceutique comprenant comme principe actif au moins une protéine constituant un canal potassium selon la revendication 1, ou un anticorps selon la revendication 2, ou une molécule d'acide nucléique selon l'une des revendications 3 ou 4 ou un vecteur selon la revendication 5.

14. Composition selon la revendication 13, **caractérisée en ce que** ledit principe actif est associé à un véhicule physiologiquement acceptable.

## Claims

1. Purified protein forming a mechanosensitive potassium channel activated by polyunsaturated fatty acids particularly arachidonic acid and by rilulozole, wherein the amino acid sequence is represented in the appended list of sequences under the number SEQ ID No:1.

2. Poly- or monoclonal antibodies targeted against at least one protein forming an ion channel according to claim 1.

3. Purified nucleic acid molecule comprising or consisting of a nucleic sequence coding for a protein according to claim 1.

4. Nucleic acid molecule according to claim 3 comprising the sequence between nucleotides 284 and 1477 of the sequence represented in the appended list of sequences under the number SEQ ID No:1 or the complementary sequence thereof.

5. Vector comprising at least one nucleic acid molecule according to any one of claims 3 or 4.

6. Method to produce a protein forming a potassium channel according to claim 1, **characterised in that** it consists of:
- transferring a nucleic acid molecule according to any one of claims 3 or 4 or a vector according to claim 5 to a cellular host,
- culturing said cellular host under conditions enabling the production of the protein forming said potassium channel,
- isolating, by any suitable means, the proteins forming said channels.

7. Method to express a potassium channel according to claim 1, **characterised in that** it consists of:
- transferring a nucleic acid molecule according to any of claims 3 or 4 or a vector according to claim 5 to a cellular host,
- culturing said cellular host under conditions enabling the production of said potassium channels.

8. Non-human cellular host liable to be obtained by means of a method according to claim 7.

9. Method to screen substances capable of modulating the activity of mechanosensitive potassium channels activated by polyunsaturated fatty acids according to claim 1, **characterised in that** variable quantities of a test substance are placed in contact with cells according to claim 7, and the potential effects of said substance on the flows of said channels are measured using any suitable means.

10. Method according to claim 9 applied to the screening of substances capable of preventing or treating diseases of the nervous system in a human or animal subject.

11. Method according to claim 10 applied to the screening of substances capable of preventing or treating neurodegeneration.

12. Method according to claim 11 applied to the screening of substances capable of preventing or treating neurodegeneration associated with ischemia and anoxia.

13. Pharmaceutical formulation comprising as its active ingredient at least one protein forming a potassium channel according to claim 1, or an antibody according to claim 2, or a nucleic acid molecule according to any of claims 3 or 4 or a vector according to claim 5.

14. Formulation according to claim 13, **characterised in that** said active ingredient is associated with a physiologically acceptable vehicle.

## Patentansprüche

1. Gereinigtes Protein, einen durch mehrfach ungesättigte Fettsäuren, vor allem die Arachidonsäure und das Riluzol, aktivierten mechanisch sensiblen Kaliumkanal ausbildend, dessen Aminosäuresequenz in der als Anlage beigefügten Sequenzliste unter der Nummer SEQ ID No:1 dargestellt ist.

2. Poly- oder monoklonale Antikörper, die gegen mindestens ein Protein gerichtet sind, die einen Ionenkanal nach Anspruch 1 ausbilden.

3. Gereinigtes Nukleinsäuremolekül, das eine für ein Protein nach Anspruch 1 kodierende Nukleinsequenz aufweist oder von ihr gebildet wird.

4. Nukleinsäuremolekül nach Anspruch 3, die Sequenz zwischen den Nukleotiden 284 und 1477 der in der als Anlage beigefügten Sequenzliste unter der Nummer SEQ ID No:1 dargestellten Sequenz aufweisend oder ihre komplementäre Sequenz.

5. Vektor, mindestens ein Nukleinsäuremolekül nach einem der Ansprüche 3 oder 4 aufweisend.

6. Verfahren zur Herstellung eines Eiweißes, das einen Kaliumkanal nach Anspruch 1 ausbildet, **dadurch gekennzeichnet, dass** es darin besteht:
- ein Nukleinsäuremolekül nach einem der Ansprüche 3 oder 4 oder einen Vektor nach Anspruch 5 in eine Wirtszelle zu transferieren,
- die besagte Wirtszelle unter solchen Bedingungen zu kultivieren, welche die Herstellung des den besagten Kaliumkanal ausbildenden Proteins erlauben,
- die besagten Kanäle ausbildenden Proteine mit allen geeigneten Mitteln zu isolieren.

7. Verfahren zur Expression eines Kaliumkanals nach Anspruch 1, **dadurch gekennzeichnet, dass** es darin besteht:
- ein Nukleinsäuremolekül nach einem der Ansprüche 3 oder 4 oder einen Vektor nach Anspruch 5 in eine Wirtszelle zu transferieren,
- die besagte Wirtszelle unter solchen Bedingungen zu kultivieren, welche die Herstellung der besagten Kaliumkanäle erlauben.

8. Nicht menschliche Wirtszelle, die nach einem Verfahren nach Anspruch 7 gewonnen werden kann.

9. Verfahren zur Auslese von Substanzen, welche die Aktivität mechanisch sensibler Kaliumkanäle, die von den mehrfach ungesättigten Fettsäuren nach Anspruch 1 aktiviert wurden, modulieren können, **dadurch gekennzeichnet, dass** variable Mengen einer zu testenden Substanz mit Zelle nach Anspruch 7 in Kontakt gebracht und dann mit allen geeigneten Mitteln die eventuellen Wirkungen der besagten Substanz auf die Strömungen der besagten Kanäle messen werden.

10. Verfahren nach Anspruch 9, angewendet auf die Auslese von Substanzen, die Krankheiten des Nervensystems beim Menschen oder Tier vorbeugen oder behandeln können.

11. Verfahren nach Anspruch 10, angewendet auf die Auslese von Substanzen, die Neurodegenerationen vorbeugen oder behandeln können.

12. Verfahren nach Anspruch 11, angewendet auf die Auslese von Substanzen, die mit Ischämien oder Anoxien verbundenen Neurodegenerationen vorbeugen oder behandeln können.

13. Pharmazeutische Zusammensetzung, mindestens ein einen Kaliumkanal nach Anspruch 1 ausbildendes Protein aufweisend, oder einen Antikörper nach Anspruch 2, oder ein Nukleinsäuremolekül nach einem der Ansprüche 3 oder 4 oder einen Vektor nach Anspruch 5.

14. Zusammensetzung nach Anspruch 13, **dadurch gekennzeichnet, dass** das besagte aktive Prinzip mit einem physiologisch akzeptablen Überträger verbunden ist.


### LISTE DE SÉQUENCES

(1) INFORMATION GÉNÉRALES:
   (iii) NOMBRE DE SEQUENCES: 2
(2) INFORMATION POUR LA SEQ ID NO:1 :
   (i) CARACTRERISTIQUES DE LA SEQUENCE:
      (A) LONGUEUR: 1794 paires de bases
      (B) TYPE: nucléotide
      (C) NOMBRE DE BRIN: double
      (D) CONFIGURATION: linéaire
   (ii) TYPE DE MOLECULE: ADN
   (ix) CARACTERISTIQUES
      (A) NOM/CLE: TRAAK
      (B) EMPLACEMENT: de 284 à 1477
   (xi) DESCRIPTION DE LA SEQUENCES: SEQ ID NO:1 :
   CCACGCGTCC GCGGACGCGT GGGTCGCCCA CGCGTCCGGT GGCGGCTGTC CTGAGCCCCG      60
   GGCCAGCTGA TGTCCAGGTT AGGGCAGCGT TGGGGCCCCA ATCCCAGCCT GGAAGGTTGG     120
   ACTTCACGTC GACCCTTCTC TGAGTCTTCT GCCACTCACT GGCCTGGACA AGACAGCATT     180
   GGGGAGCCCA GAGGCTGCAG GTGCAGTGAC CACTGCTCCC CAGGAGCTCC CTGCTCCTTC     240
   TTCCCAGGCA GGAAGTGGAG CTGGACCTGC CTCTGGAAGG ACC ATG CGC AGC ACC       295
                                                   Met Arg Ser Thr
                                                   1
   ACA CTC CTG GCT CTG CTG GCA CTG GTG CTG CTT TAC TTG GTA TCT GGG       343
   Thr Leu Leu Ala Leu Leu Ala Leu Val Leu Leu Tyr Leu Val Ser Gly
   5                   10                  15                  20
   GCT CTA GTG TTC CAG GCT CTG GAG CAG CCT CAC GAG CAG CAG GCT CAG       391
   Ala Leu Val Phe Gln Ala Leu Glu Gln Pro His Glu Gln Gln Ala Gln
                   25                  30                  35
   AAG AAA ATG GAT CAT GGC CGA GAC CAG TTT CTG AGG GAC CAT CCC TGT       439
   Lys Lys Met Asp His Gly Arg Asp Gln Phe Leu Arg Asp His Pro Cys
               40                  45                  50
   GTG AGC CAG AAG AGC CTG GAG GAT TTC ATC AAG CTC CTG GTT GAA GCC       487
   Val Ser Gln Lys Ser Leu Glu Asp Phe Ile Lys Leu Leu Val Glu Ala
           55                  60                  65
   CTG GGA GGG GGC GCA AAC CCA GAA ACC AGC TGG ACC AAT AGC AGC AAC       535
   Leu Gly Gly Gly Ala Asn Pro Glu Thr Ser Trp Thr Asn Ser Ser Asn
       70                  75                  80
   CAC TCA TCA GCT TGG AAC CTG GGC AGC GCC TTC TTT TTC TCG GGG ACC       583
   His Ser Ser Ala Trp Asn Leu Gly Ser Ala Phe Phe Phe Ser Gly Thr
   85                  90                  95                  100
   ATC ATC ACT ACC ATC GGC TAT GGC AAT ATA GTC TTA CAC ACA GAT GCC       631
   Ile Ile Thr Thr Ile Gly Tyr Gly Asn Ile Val Leu His Thr Asp Ala
                   105                 110                 115
   GGG CGT CTC TTT TGT ATC TTC TAT GCA CTG GTG GGG ATC CCA CTG TTC      679
   Gly Arg Leu Phe Cys Ile Phe Tyr Ala Leu Val Gly Ile Pro Leu Phe
               120                 125                 130
   GGG ATG CTG CTG GCG GGA GTC GGG GAC CGG CTG GGC TCC TCT CTG CGC      727
   Gly Met Leu Leu Ala Gly Val Gly Asp Arg Leu Gly Ser Ser Leu Arg
           135                 140                 145
   CGG GGC ATC GGC CAC ATC GAA GCA ATC TTC TTG AAG TGG CAT GTG CCA      775
   Arg Gly Ile Gly His Ile Glu Ala Ile Phe Leu Lys Trp His Val Pro
       150                 155                 160
   CCG GGG CTG GTG AGA AGT CTG TCC GCA GTG CTC TTC CTG CTG ATC GGC      823
   Pro Gly Leu Val Arg Ser Leu Ser Ala Val Leu Phe Leu Leu Ile Gly
   165                 170                 175                 180
   TGC CTG CTC TTT GTC CTC ACT CCT ACC TTC GTG TTC TCC TAC ATG GAG      871
   Cys Leu Leu Phe Val Leu Thr Pro Thr Phe Val Phe Ser Tyr Met Glu
                   185                 190                 195
   AGC TGG AGC AAG TTA GAA GCC ATC TAC TTT GTT ATA GTG ACT CTC ACC      919
   Ser Trp Ser Lys Leu Glu Ala Ile Tyr Phe Val Ile Val Thr Leu Thr
               200                 205                 210
   ACT GTA GGC TTT GGC GAT TAT GTA CCC GGC GAT GGC ACC GGG CAG AAC      967
   Thr Val Gly Phe Gly Asp Tyr Val Pro Gly Asp Gly Thr Gly Gln Asn
           215                 220                 225
   TCT CCA GCC TAC CAG CCG CTG GTG TGG TTC TGG ATC TTG TTT GGC CTA     1015
   Ser Pro Ala Tyr Gln Pro Leu Val Trp Phe Trp Ile Leu Phe Gly Leu
       230                 235                 240
   GCC TAC TTC GCC TCA GTG CTC ACC ACC ATC GGC AAC TGG TTG CGA GCA     1063
   Ala Tyr Phe Ala Ser Val Leu Thr Thr Ile Gly Asn Trp Leu Arg Ala
   245                 250                 255                 260
   GTG TCC CGC CGA ACT CGG GCA GAG ATG GGT GGC CTA ACG GCA CAG GCT     1111
   Val Ser Arg Arg Thr Arg Ala Glu Met Gly Gly Leu Thr Ala Gln Ala
                   265                 270                 275
   GCT AGC TGG ACC GGC ACA GTG ACA GCG CGA GTG ACC CAG CGA ACT GGG     1159
   Ala Ser Trp Thr Gly Thr Val Thr Ala Arg Val Thr Gln Arg Thr Gly
               280                 285                 290
   CCC AGC GCC CCG CCG CCA GAG AAG GAG CAA CCA CTC CTG CCC TCC TCT     1207
   Pro Ser Ala Pro Pro Pro Glu Lys Glu Gln Pro Leu Leu Pro Ser Ser
           295                 300                 305
   TTG CCG GCA CCG CCT GCT GTT GTT GAG CCA GCC GGC AGG CCC GGC TCC     1255
   Leu Pro Ala Pro Pro Ala Val Val Glu Pro Ala Gly Arg Pro Gly Ser
       310                 315                 320
   CCT GCA CCC GCA GAG AAG GTT GAG ACT CCG TCC CCG CCC ACG GCC TCA     1303
   Pro Ala Pro Ala Glu Lys Val Glu Thr Pro Ser Pro Pro Thr Ala Ser
   325                 330                 335                 340
   GCT CTG GAT TAC CCC AGT GAG AAT CTG GCC TTC ATC GAC GAG TCC TCA     1351
   Ala Leu Asp Tyr Pro Ser Glu Asn Leu Ala Phe Ile Asp Glu Ser Ser
                   345                 350                 355
   GAC ACG CAG AGT GAG CGT GGC TGT GCC CTG CCT CGG GCT CCT CGG GGT     1399
   Asp Thr Gln Ser Glu Arg Gly Cys Ala Leu Pro Arg Ala Pro Arg Gly
               360                 365                 370
   CGC CGC CGA CCC AAC CCA TCC AAA AAG CCT TCC AGA CCC CGG GGT CCT     1447
   Arg Arg Arg Pro Asn Pro Ser Lys Lys Pro Ser Arg Pro Arg Gly Pro
           375                 380                 385
   GGG CGA CTC CGA GAC AAG GCC GTG CCG GTG TAG GGGCAGGATC              1490
   Gly Arg Leu Arg Asp Lys Ala Val Pro Val  *
       390                 395         398
   TCTGGACCCG GATCCCACGC CAGGGCTTTC GCTCTTGCTG ATGCTCAGGC ATGCTTGGCT   1550
   TATTTGACCA AAGAGCCGTC CCTCTTTTGT TCCACGTGGT TGCAACCCTG ACAGGAGTCC   1610
   AGTGGTTGCC AAATGCCACC GCTCTTCCCT GGCTGGTTCT TCACATCCAA TCATTTCCAA   1670
   AGCCCACCAT CCAAGGCTTT CTGCCTCGCT CCCCTGCCGG TTTTGACCCT CACACCTCAC   1730
   AACTGTGCCT CAAAACCTGC ACCAATAAAA CAAAAACTCT GAAAAAAAAA AAAAAAAAAA   1790
   AAAA                                                                1794
(2) INFORMATION POUR LA SEQ ID NO: 2 :
   (i) CARACTRERISTIQUES DE LA SEQUENCE:
      (A) LONGUEUR: ... paires de bases
      (B) TYPE: nucléotide
      (C) NOMBRE DE BRIN: double
      (D) CONFIGURATION: linéaire
   (ii) TYPE DE MOLECULE: ADN
   (ix) CARACTERISTIQUES
      (A) NOM/CLE: TREK-1

      (B) EMPLACEMENT: de 484 à 1596
   (xi) DESCRIPTION DE LA SEQUENCES: SEQ ID NO:2